# EUROPEAN PATENT SPECIFICATION

(11) **EP 4 518 823 B1**
(45) Date of publication and mention of the grant of the patent: **03.12.2025**
(21) Application number: 23704838.4
(22) Date of filing: 27.01.2023
(51) Int. Cl.: A61G 11/00

(54) **AN INFANT INCUBATOR**
INKUBATOR FÜR KLEINKINDER
COUVEUSE POUR NOURRISSON

(30) Priority: 06.05.2022 LV 220039
(43) Date of publication of application: 12.03.2025
(73) Proprietor: Armgate, Sia, Marupe 2167 (LV)
(72) Inventor: VARDANJANS, Reviks, 2010 Jurmala (LV)
(74) Representative: Kromanis, Artis
(86) International application number: PCT/LV2023/050002
(87) International publication number: WO 2023/214865

(56) References cited:
- US-A- 4 750 474
- US-A- 5 100 375
- US-A- 5 616 115

## Description

### Field of the invention

The present invention relates to infant incubators.

### Background of the invention

The prior art discloses various infant incubators. The U.S. patent publication No. US5,100,375 discloses an incubator for infants comprising a hood and a infant compartment enclosed by the hood and wherein air is supplied from an air supply opening arranged between the side walls of the incubator hood and a supporting member defining a supporting surface for accommodating the infant. This incubator is one wall hood incubator with only some parts of the hood to be two walled constructions. Moreover, a pipe is installed into the infant compartment and wherein the pipe is used for collecting the air from an incubator. The U.S patent application publication No. US2015/0065788 discloses an incubator with a double glazed wall. The inner and external glasses are spaced by a spacer, and are characterized by an inner atmosphere confined within the canopy and an external atmosphere.

The U.S. patent publication No. US4,936,824 discloses an infant incubator which comprises a base, an infant compartment and a hood that encloses the infant compartment. Moreover, the hood is made as the double wall hood allowing a warming air flow re-circulate around the infant compartment. Moreover, the incubator further comprises air curtain. Similar design is disclosed in European patent application publication No. EP0931534.

European patent application publication No. EP0032133 discloses an incubator for infants comprising a base and a partly cylindrical hood enclosing an incubator compartment. Heating of the incubator compartment is achieved by circulating air through a double wall construction of the hood. Similar design is disclosed in the U.S. patent publication No. US4,750,474.

It is essential to maintain the necessary temperature within the infant incubator. This is not always a case when big temperature difference is present between the inner atmosphere and the external atmosphere. Accordingly, it would be advantageous to design an infant incubator allowing to sustain the necessary temperature within the infant compartment mitigating influence of the external atmosphere.

### Summary of the invention

The invention is disclosed in the claims.

The present invention provides an improved infant incubator having means to allow to sustain the necessary temperature within the inner atmosphere mitigating influence of the external atmosphere.

The aim is reached by a design of an infant incubator comprising a base, a mattress arranged on the base and adapted to support an infant, a hood and an access door. The present invention is characterized in that the hood is a triple walled hood and the access door is a triple walled access door. The triple walled hood surrounding said base to enclose an infant compartment therebetween for containing an infant. The triple walled access door is pivotally mounted to the base.

The triple walled hood and the triple walled access door comprises an inner wall, an intermediate wall and an external wall spaced apart from each other so that an inner space is formed between the inner wall and the intermediate wall and an outer space is formed between the intermediate wall and the external wall. The inner space and the outer space are fluidly connected to each other by means of an opening or openings. The amount and size of an opening and openings are configured based on necessary warm gas flow for necessary incubator volume or infant compartment volume. The inner wall completely seals the infant compartment from the spaces of the triple walled hood and the spaces of the triple walled access door. In result of this an independent air or gas circulation can be provided within the infant compartment and within the triple walled hood and the triple walled access door. This allows to supply an air to the infant compartment specifically suitable for the infant. The air or gas supply in the triple walled hood and in the triple walled access door is specifically adapted for heating purposes in order to maintain a necessary or set temperature within the infant compartment. In other words, the inner wall and the external wall encloses the spaces so that said spaces are completely sealed from the infant compartment and external environment, for example a room in which the incubator is located. This non-existent fluid communication of the spaces with the infant compartment and the external environment allows more energy-effective and agile control or warming of the infant compartment.

The infant incubator comprises a gas flow preparation unit configured to heat and provide a warm gas flow via inlets to the inner space of the triple walled hood and to the inner space of the triple walled access door and further through the openings into the outer space of the triple walled hood and into the outer space of the triple walled access door respectively and further out from said outer spaces via outlets and back to the gas flow preparation unit forming a closed loop of the warm gas flow, in result of which effective heating of the infant compartment is provided. The outer space acts like an insulation to the inner space - less heated, warmed gas flow may be provided maintaining the same characteristics as it would be with double walled incubators. Hence, the present invention is not only more energy efficient but also provides less dangerous (heated) inner wall, which can harm the infant within the incubator.

The gas flow preparation unit comprises a heating means. The heating means may be selected from a group consisting of: gas-conditioned system, an infrared heater, a water/oil heated radiator, a coiled heater, an open coil gas heater, a round open coil gas heater, a convection heater, straight and formed tubular heater, a quartz tube gas heater, a capacitor-type heater, thermoelectric heat-pump. The gas flow is provided by a fan driven by electricity. Any gas flow preparation unit for infant incubators known to the skilled person may be used.

Gas to be warmed and circulated is selected from the group comprising air, xenon, oxygen, nitrogen, carbon dioxide, helium, or a mixture thereof.

The triple walled access door is pivotally mounted to the base by means of a tube hinge which is a part of a tube for providing the warm gas flow from the gas flow preparation unit to the inlets and from the outlets back to the gas flow preparation unit. This tube hinge allows to provide continuous gas flow not only when the door is in closed position but also when the door is in open position. Therefore, there is no need for additional warming up of the door when it has been opened.

The triple walled hood and the triple walled access door are made of transparent material. The wall of the triple walled hood and the triple walled access door may be made of glass, polymers or combinations thereof. For the safety reasons the wall may be laminated so that in the event of breaking it is held in place by said lamination.

In other embodiment of the invention the infant incubator may comprise a triple walled base floor built under the mattress of the incubator. The triple walled base floor comprises an inner wall, an intermediate wall and an external wall spaced apart from each other so that an inner space is formed between the inner wall and the intermediate wall, and an outer space is formed between the intermediate wall and the external wall. The inner space and the outer space are fluidly connected to each other by means of openings. The inner space and the outer space of the triple walled base floor are fluidly connected to the gas flow preparation unit in the same way as it is for the triple walled hood and the triple walled access door.

The inner space of the triple walled hood, the triple walled access door and the triple walled base floor is positioned closer to the infant compartment to be warmed than the outer space of the triple walled hood, the triple walled access door and the triple walled base floor.

The triple walled hood, the triple walled access door and the triple walled base floor may be covered with metallized or electro conductive layer to control heat radiation and to provide localized heating.

The space between the walls of the triple walled hood, the triple walled access door and the triple walled base floor may be filled with or contain desiccant to remove moisture. The desiccant is arranged within the first space and/or the second space so that it does not obstruct the gas flow within the same space.

The gas flow preparation unit provides the warm gas flow to the triple walled hood and separately to the triple walled access door and triple walled base floor so that the infant incubator comprises three separate, independent closed loop warm gas circuits.

### Brief description of the drawings

The drawings illustrate generally, by way of example, but not by way of limitation, various embodiments of the invention.
Fig. 1 is a perspective view of an infant incubator (1) with slightly opened triple walled access doors (6).
Fig. 2 is a side view of the infant incubator (1) as seen in Fig. 1.
Fig. 3 is a cross-section view of a triple walled hood (4), a triple walled access door (6) and a triple walled base floor (20) as seen in A-A of Fig. 2.
Fig. 4 is a cross-section view of a triple walled hood (4) and a triple walled access door (6) as seen in C-C of Fig. 2.
Fig. 5 is a back view of the infant incubator (1) as seen in Figs. 1 and 2 with principial representation of a gas flow preparation unit (13).
Fig. 6 is a cross-section view of a triple walled hood (4) and a triple walled base floor (20) as seen in B-B of Fig. 5.
Fig 7. is a perspective view of an infant incubator (1) with removed triple walled hood (4) and triple walled access door (6).
Fig 8. is a top view of the infant incubator (1) as seen in Fig. 7.
Fig. 9 is a cross-section view of the base (2) of the infant incubator (1) as seen in A-A of Fig. 8.
Fig. 10 is a cross-section view of the base (2) of the infant incubator (1) as seen in B-B of Fig. 8.
Fig. 11 is a cross-section view of the base (2) of the infant incubator (1) as seen in C-C of Fig. 8.
Fig. 12 is a perspective view of another embodiment of the present invention.

### Detailed description of the embodiments

The preferred embodiments of the invention are now described with reference to the figures to illustrate objectives, advantages, and efficiency of the present invention.

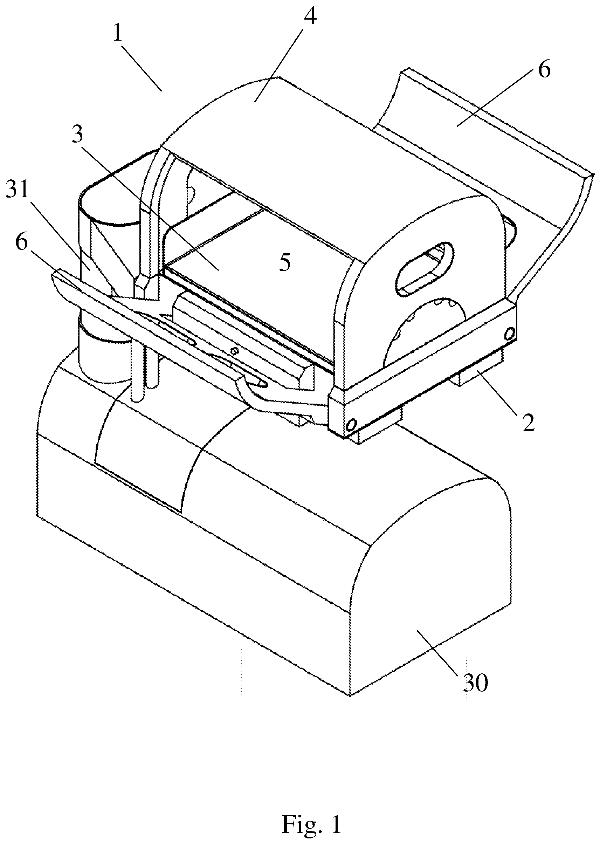

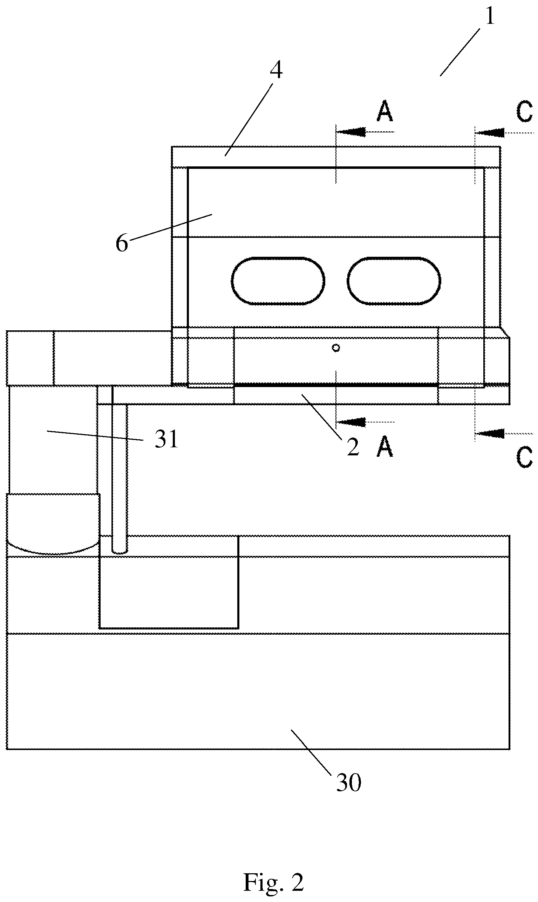

Fig. 1 is a perspective view of an infant incubator (1) and Fig. 2 illustrates the same infant incubator (1) from one of its sides. The infant incubator (1) comprises a base (2). In the present embodiment the base (2) is attached to a column (31) of an incubator carrier (30). The infant incubator (1) comprises a mattress (3) arranged on the base (2) and adapted to support an infant. The infant incubator (1) comprises a triple walled hood (4) surrounding said base (2) to enclose an infant compartment (5) therebetween for containing an infant, and a triple walled access door (6) pivotally mounted to the base (2) further enclosing the infant compartment (5) and at the same time providing access to the infant compartment (5). The triple walled access doors (6) are slightly opened.

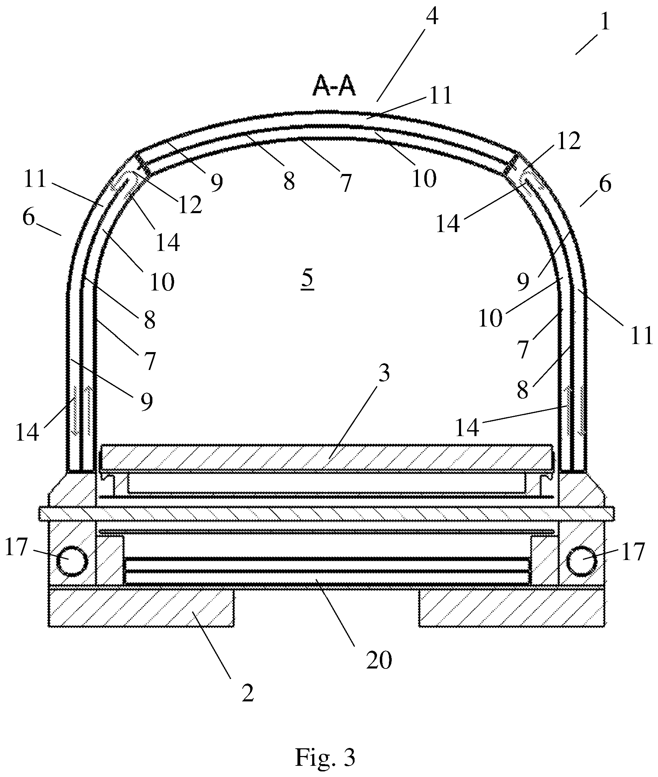

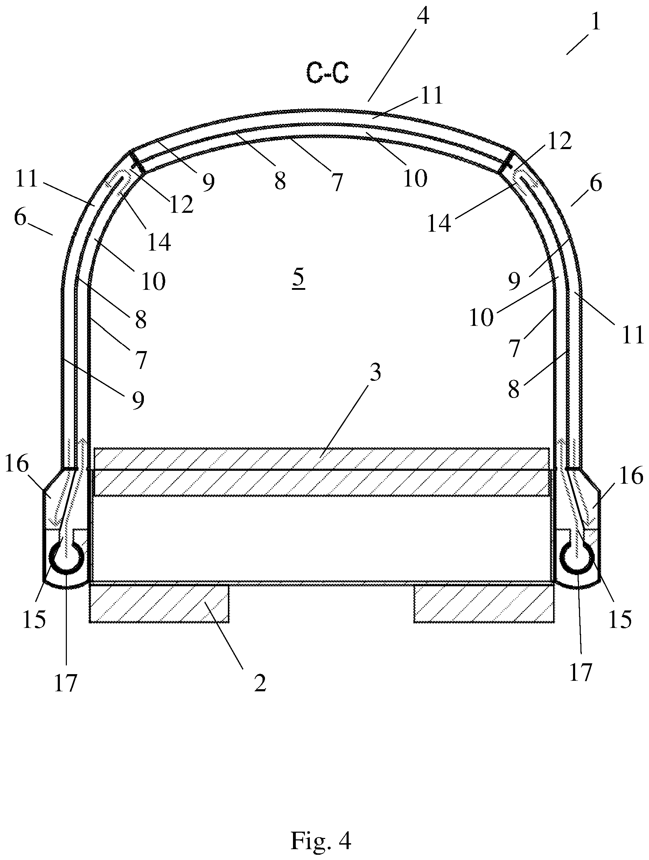

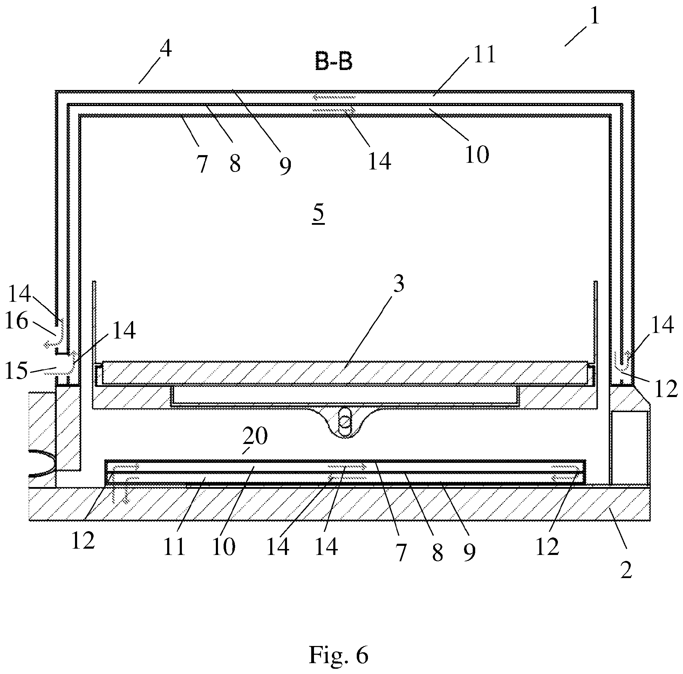

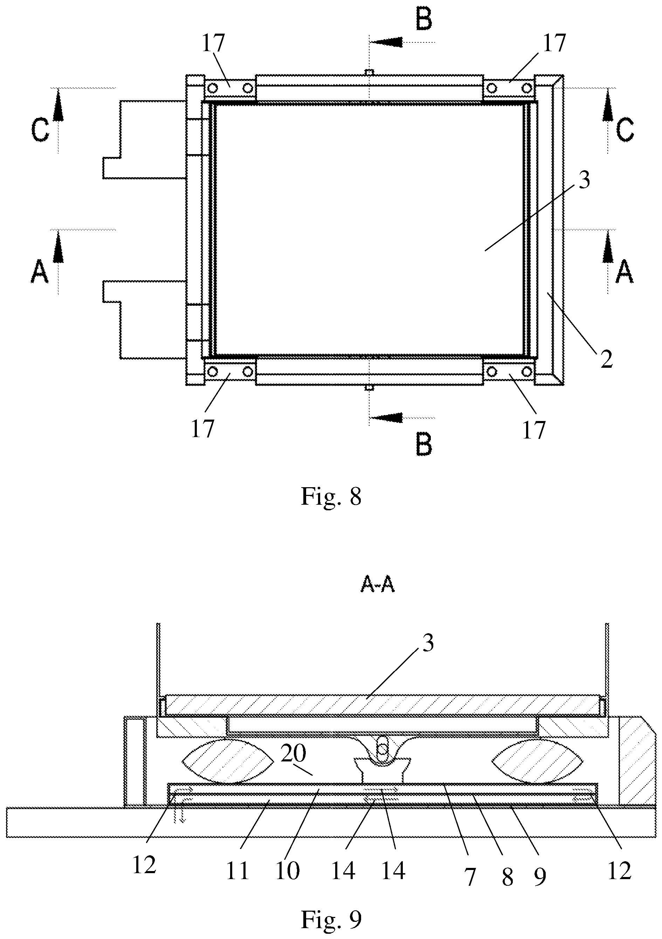

Fig. 3 is a cross-section view of a triple walled hood (4), a triple walled access door (6) and a triple walled base floor (20) as seen in A-A of Fig. 2 and Fig. 4 is a cross-section view of a triple walled hood (4) and a triple walled access door (6) as seen in C-C of Fig. 2 (The triple walled base floor (20) is seen in more detail in Figs. 6, 9 and 10). The triple walled hood (4) and the triple walled access door (6) comprises an inner wall (7), an intermediate wall (8) and an external wall (9). The inner wall (7) and the intermediate wall (8) are spaced apart from each other so that an inner space (10) is formed therebetween. The intermediate wall (8) and the external wall (9) are spaced apart from each other so that an outer space (11) is formed therebetween. The inner space (10) and the outer space (11) are fluidly connected to each other by means of openings (12). The inner space (10) of the triple walled hood (4) and the triple walled access door (6) is positioned closer to the infant compartment (5) to be warmed than the outer space (11) of the triple walled hood (4) and the triple walled access door (6).

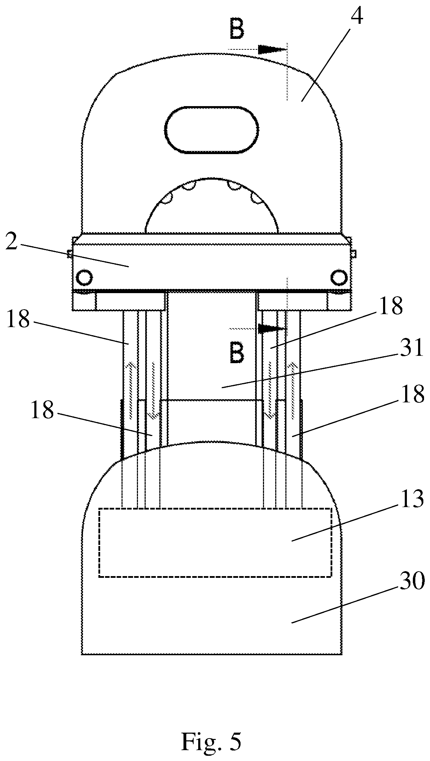

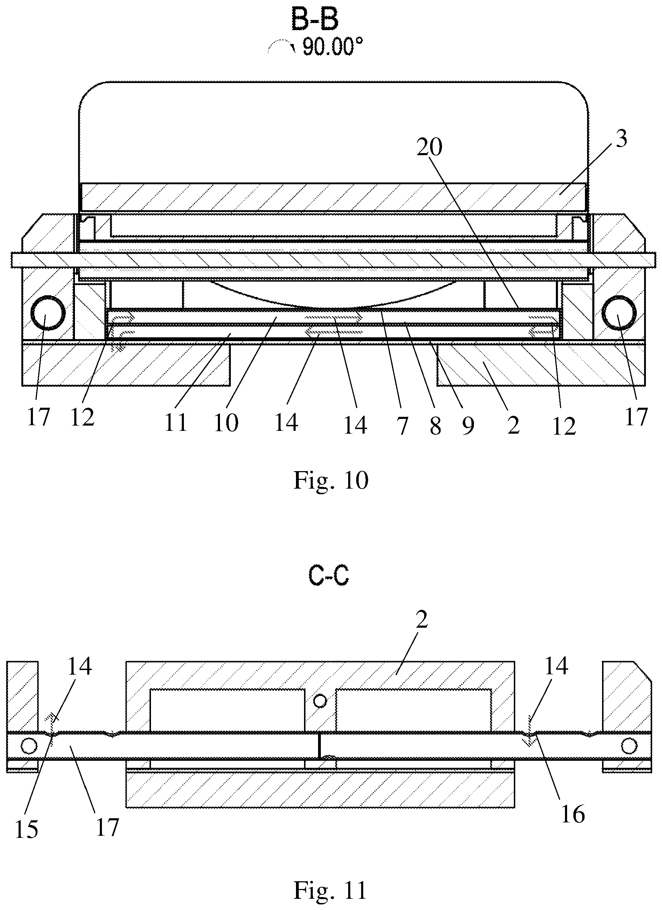

The infant incubator comprises a gas flow preparation unit (13) configured to heat and provide a warm gas flow (14) to the triple walled hood (4), the triple walled access door (6) and a triple walled base floor (20) (see Fig. 5). The gas flow preparation unit (13) is installed in the carrier (30) of the infant incubator (1) and the gas flow preparation unit (13) is connected to the triple walled hood (4), the triple walled access door (6) and a triple walled base floor (20) through conduits (18). The gas flow preparation unit comprises a heating means for heating the gas flow and a fan for providing said gas flow. The warm gas flow (14) is provided to the inner space (10) of the triple walled hood (4) and to the inner space (10) of the triple walled access door (6) via inlets (15) as seen in Figs. 6 and 11. The warm gas flow (14) is further conducted through the openings (12) in the intermediate wall (8) into the outer space (11) of the triple walled hood (4) and the outer space (11) of the triple walled access door (6). The warm gas flow (14) is further conducted out from said outer spaces (11) via outlets (16) and back to the gas flow preparation unit (13) forming a closed loop of the warm gas flow (14) as seen in Figs. 2, 4, 5 and 6. The warm gas flow (14) does not enter or exit the infant compartment (5), as well as does not enter or exit to an external environment. Hence, effective heating of the infant compartment (5) is provided.

The triple walled base floor (20) is seen in more detail in Figs. 6, 9 and 10. The triple walled base floor (20) comprises an inner wall (7), an intermediate wall (8) and an external wall (9) spaced apart from each other. An inner space (10) is formed between the inner wall (7) and the intermediate wall (8). An outer space (11) is formed between the intermediate wall (8) and the external wall (9). The inner space (10) and the outer space (11) are fluidly connected to each other by means of openings (12) and said inner space (10) and outer space (11) of the triple walled base floor (20) are fluidly connected to the gas flow preparation unit (13).

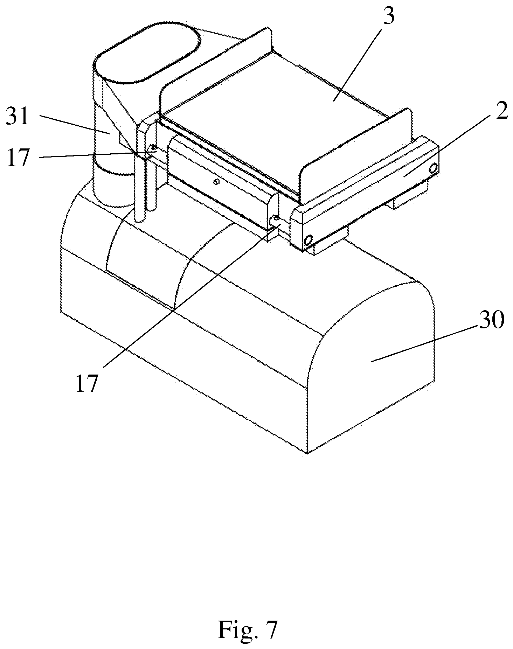

The triple walled access door (6) is pivotally mounted to the base (2) by means of a tube hinge (17) which is a part of a tube for providing the warm gas flow (14) from the gas flow preparation unit (13) to the inlets (15) and from the outlets (16) back to the gas flow preparation unit (13). See Figs. 3, 4, 7, 8, 10 and 11.

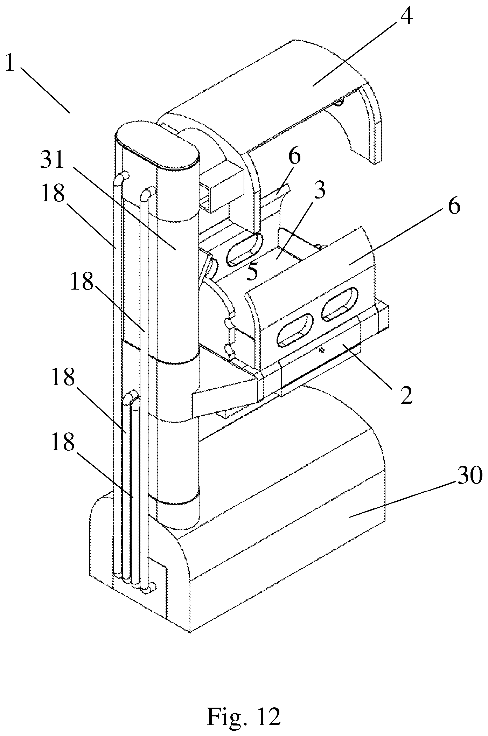

Fig. 12 is a perspective view of another embodiment of the present invention. The infant incubator (1) comprises extendable column (31) compared to the previously described embodiment allowing to lift and lower the triple walled hood (4) vertically. This design allows to remove the triple walled hood (4) and triple walled access doors (6) so that there is full, non-restricted access to the infant compartment (5).

## Claims

1. An infant incubator (1) comprising:
- a base (2),
- a mattress (3) arranged on the base (2) and adapted to support an infant,
- a triple walled hood (4) surrounding said base (2) to enclose an infant compartment (5) therebetween for containing an infant,
- a triple walled access door (6) pivotally mounted to the base (2),
- the triple walled hood (4) and the triple walled access door (6) comprises an inner wall (7), an intermediate wall (8) and an external wall (9) spaced apart from each other so that an inner space (10) is formed between the inner wall (7) and the intermediate wall (8), and an outer space (11) is formed between the intermediate wall (8) and the external wall (9), wherein the inner space (10) and the outer space (11) are fluidly connected to each other by means of openings (12) in the intermediate wall (8), wherein the inner space (10) of the triple walled hood (4) and the triple walled access door (6) is positioned closer to the infant compartment (5) to be warmed than the outer space (11) of the triple walled hood (4) and the triple walled access door (6), and wherein the inner wall (7) and the external wall (9) encloses the spaces (10, 11) so that said spaces (10, 11) are completely sealed from the infant compartment (5) and external environment,
- a gas flow preparation unit (13) configured to heat and provide a warm gas flow (14) via inlets (15) to the inner space (10) of the triple walled hood (4) and to the inner space (10) of the triple walled access door (6) and further through the openings (12) into the outer space (11) of the triple walled hood (4) and the outer space (11) of the triple walled access door (6) respectively and further out from said outer spaces (11) via outlets (16) and back to the gas flow preparation unit (13) forming a closed loop of the warm gas flow (14), in result of which effective heating of the infant compartment (5) is provided.

2. The infant incubator (1) according to Claim 1, **characterized in that** the triple walled access door (6) is pivotally mounted to the base (2) by means of a tube hinge (17) which is a part of a tube for providing the warm gas flow (14) from the gas flow preparation unit (13) to the inlets (15) and from the outlets (16) back to the gas flow preparation unit (13).

3. The infant incubator (1) according to Claim 1 or 2, **characterized in that** the base (2) comprises a triple walled base floor (20) arranged below the mattress (3), wherein the triple walled base floor (20) comprises an inner wall (7), an intermediate wall (8) and an external wall (9) spaced apart from each other so that an inner space (10) is formed between the inner wall (7) and the intermediate wall (8), and an outer space (11) is formed between the intermediate wall (8) and the external wall (9), wherein the inner space (10) and the outer space (11) are fluidly connected to each other by means of openings (12) and said inner space (10) and outer space (11) of the triple walled base floor (20) are fluidly connected to the gas flow preparation unit (13).

4. The infant incubator (1) according to any of claims 1 to 3, **characterized in that** the triple walled hood (4) and the triple walled access door (6) are made of transparent material.

5. The infant incubator (1) according to any of claims 1 to 4, **characterized in that** the triple walled hood (4), the triple walled access door (6) and the triple walled base floor (20) is covered with metallized or electroconductive layer.

6. The infant incubator (1) according to any of claims 1 to 5, **characterized in that** the gas flow preparation unit (13) provides the warm gas flow (14) to the triple walled hood (4) and separately to the triple walled access door (6) and triple walled base floor (20) so that the infant incubator (1) comprises three separate closed loop warm gas circuits.

## Patentansprüche

1. Ein Säuglingsinkubator (1), bestehend aus:
- einer Basis (2),
- eine auf der Basis (2) angeordnete Matratze (3), die zum Tragen eines Säuglings ausgelegt ist,
- eine dreifachwandige Haube (4), die die Basis (2) umgibt, um dazwischen einen Säuglingsraum (5) zur Aufnahme eines Säuglings zu bilden,
- eine dreifachwandige Zugangstür (6), die schwenkbar an der Basis (2) angebracht ist,
- wobei die dreifachwandige Haube (4) und die dreifachwandige Zugangstür (6) eine Innenwand (7), eine Zwischenwand (8) und eine Außenwand (9) umfassen, die voneinander beabstandet sind, so dass zwischen der Innenwand (7) und der Zwischenwand (8) ein Innenraum (10) gebildet wird, und ein Außenraum (11) zwischen der Zwischenwand (8) und der Außenwand (9) gebildet wird, wobei der Innenraum (10) und der Außenraum (11) durch Öffnungen (12) in der Zwischenwand (8) fluidisch miteinander verbunden sind, wobei der Innenraum (10) der dreifachwandigen Haube (4) und der dreifachwandigen Zugangstür (6) näher an dem zu erwärmenden Säuglingsraum (5) angeordnet ist als der Außenraum (11) der dreifachwandigen Haube (4) und der dreifachwandigen Zugangstür (6), und wobei die Innenwand (7) und die Außenwand (9) die Räume (10, 11) so umschließen, dass die Räume (10, 11) vollständig vom Säuglingsraum (5) und der Außenumgebung abgedichtet sind,
- eine Gasstromvorbereitungseinheit (13), die so konfiguriert ist, dass sie einen warmen Gasstrom (14) erwärmt und über Einlässe (15) in den Innenraum (10) der dreifachwandigen Haube (4) und in den Innenraum (10) der dreifachwandigen Zugangstür (6) und weiter durch die Öffnungen (12) in den Außenraum (11) der dreifachwandigen Haube (4) und den Außenraum (11) der dreifachwandigen Zugangstür (6) zu leiten und weiter aus den Außenräumen (11) über Auslässe (16) zurück zur Gasstromvorbereitungseinheit (13) zu leiten, wodurch ein geschlossener Kreislauf des warmen Gasstroms (14) gebildet wird, wodurch eine wirksame Beheizung des Säuglingsabteils (5) bereitgestellt wird.

2. Der Säuglingsinkubator (1) nach Anspruch 1, **dadurch gekennzeichnet, dass** die dreifachwandige Zugangstür (6) mittels eines Rohrscharniers (17), das Teil eines Rohrs zur Bereitstellung des Warmgasstroms (14) von der Gasstromaufbereitungseinheit (13) zu den Einlässen (15) und von den Auslässen (16) zurück zur Gasstromaufbereitungseinheit (13) dient.

3. Der Säuglingsinkubator (1) nach Anspruch 1 oder 2, **dadurch gekennzeichnet, dass** die Basis (2) einen dreifachwandigen Basisboden (20) umfasst, der unterhalb der Matratze (3) angeordnet ist, wobei der dreifachwandige Basisboden (20) eine Innenwand (7), eine Zwischenwand (8) und eine Außenwand (9) umfasst, die voneinander beabstandet sind, so dass zwischen der Innenwand (7) und der Zwischenwand (8) ein Innenraum (10) und zwischen der Zwischenwand (8) und der Außenwand (9) einen Außenraum (11) gebildet wird, wobei der Innenraum (10) und der Außenraum (11) mittels Öffnungen (12) fluidisch miteinander verbunden sind und der Innenraum (10) und der Außenraum (11) des dreifachwandigen Bodenbodens (20) fluidisch mit der Gasstromaufbereitungseinheit (13) verbunden sind.

4. Der Säuglingsinkubator (1) nach einem der Ansprüche 1 bis 3, **dadurch gekennzeichnet, dass** die dreifachwandige Haube (4) und die dreifachwandige Zugangstür (6) aus transparentem Material bestehen.

5. Der Säuglingsinkubator (1) nach einem der Ansprüche 1 bis 4, **dadurch gekennzeichnet, dass** die dreifachwandige Haube (4), die dreifachwandige Zugangstür (6) und der dreifachwandige Boden (20) mit einer metallisierten oder elektrisch leitfähigen Schicht bedeckt sind.

6. Der Säuglingsinkubator (1) nach einem der Ansprüche 1 bis 5, **dadurch gekennzeichnet, dass** die Gasstromaufbereitungseinheit (13) den warmen Gasstrom (14) der dreifachwandigen Haube (4) und separat der dreifachwandigen Zugangstür (6) und dem dreifachwandigen Boden (20) zuführt, so dass der Säuglingsinkubator (1) drei separate geschlossene Warmgas-Kreisläufe umfasst.

## Revendications

1. Incubateur pour nourrissons (1) comprenant:
- une base (2),
- un matelas (3) disposé sur la base (2) et adapté pour supporter un nourrisson,
- un capot à triple paroi (4) entourant ladite base (2) afin d'enfermer entre eux un compartiment pour nourrisson (5) destiné à contenir un nourrisson,
- une porte d'accès à triple paroi (6) montée de manière pivotante sur la base (2),
- la hotte à triple paroi (4) et la porte d'accès à triple paroi (6) comprennent une paroi intérieure (7), une paroi intermédiaire (8) et une paroi extérieure (9) espacées les unes des autres de manière à former un espace intérieur (10) entre la paroi intérieure (7) et la paroi intermédiaire (8), et un espace extérieur (11) est formé entre la paroi intermédiaire (8) et la paroi extérieure (9), dans lequel l'espace intérieur (10) et l'espace extérieur (11) sont reliés de manière fluide l'un à l'autre au moyen d'ouvertures (12) dans la paroi intermédiaire (8), dans lequel l'espace intérieur (10) de la hotte à triple paroi (4) et la porte d'accès à triple paroi (6) est positionné plus près du compartiment pour nourrissons (5) à réchauffer que l'espace extérieur (11) de la hotte à triple paroi (4) et de la porte d'accès à triple paroi (6), et dans lequel la paroi intérieure (7) et la paroi extérieure (9) entourent les espaces (10, 11) de telle sorte que lesdits espaces (10, 11) sont complètement isolés du compartiment pour nourrissons (5) et de l'environnement extérieur,
- une unité de préparation du flux de gaz (13) configurée pour chauffer et fournir un flux de gaz chaud (14) via des entrées (15) vers l'espace intérieur (10) de la hotte à triple paroi (4) et vers l'espace intérieur (10) de la porte d'accès à triple paroi (6), puis à travers les ouvertures (12) vers l'espace extérieur (11) de la hotte à triple paroi (4) et l'espace extérieur (11) de la porte d'accès à triple paroi (6) respectivement, puis hors desdits espaces extérieurs (11) via des sorties (16) et de retour vers l'unité de préparation du flux de gaz (13), formant ainsi une boucle fermée du flux de gaz chaud (14), ce qui permet un chauffage efficace du compartiment pour nourrissons (5).

2. L'incubateur pour nourrissons (1) selon la revendication 1, **caractérisé en ce que** la porte d'accès à triple paroi (6) est montée de manière pivotante sur la base (2) au moyen d'une charnière tubulaire (17) qui fait partie d'un tube destiné à fournir le flux de gaz chaud (14) depuis l'unité de préparation du flux de gaz (13) vers les entrées (15) et des sorties (16) vers l'unité de préparation du flux de gaz (13).

3. L'incubateur pour nourrissons (1) selon la revendication 1 ou 2, **caractérisé en ce que** la base (2) comprend un fond de base à triple paroi (20) disposé sous le matelas (3), dans lequel le fond de base à triple paroi (20) comprend une paroi intérieure (7), une paroi intermédiaire (8) et une paroi externe (9) espacées les unes des autres de manière à former un espace intérieur (10) entre la paroi intérieure (7) et la paroi intermédiaire (8), et un espace extérieur (11) entre la paroi intermédiaire (8) et la paroi externe (9), dans lequel l'espace intérieur (10) et l'espace extérieur (11) sont reliés de manière fluide l'un à l'autre au moyen d'ouvertures (12) et ledit espace intérieur (10) et ledit espace extérieur (11) du fond à triple paroi (20) sont reliés de manière fluide à l'unité de préparation du flux de gaz (13).

4. L'incubateur pour nourrissons (1) selon l'une quelconque des revendications 1 à 3, **caractérisé en ce que** la hotte à triple paroi (4) et la porte d'accès à triple paroi (6) sont réalisées en un matériau transparent.

5. L'incubateur pour nourrissons (1) selon l'une quelconque des revendications 1 à 4, **caractérisé en ce que** la hotte à triple paroi (4), la porte d'accès à triple paroi (6) et le plancher de base à triple paroi (20) sont recouverts d'une couche métallisée ou électroconductrice.

6. Incubateur pour nourrissons (1) selon l'une quelconque des revendications 1 à 5, **caractérisé en ce que** l'unité de préparation du flux de gaz (13) fournit le flux de gaz chaud (14) à la hotte à triple paroi (4) et séparément à la porte d'accès à triple paroi (6) et au plancher de base à triple paroi (20), de sorte que l'incubateur pour nourrissons (1) comprend trois circuits de gaz chaud en boucle fermée séparés.
